# EUROPEAN PATENT APPLICATION

(11) **EP 4 230 159 A1**
(43) Date of publication of application: **23.08.2023**
(21) Application number: 23171885.9
(22) Date of filing: 22.06.2016
(51) Int. Cl.: A61B 17/80, A61B 17/064

(54) **BONE PLATE AND BONE SYSTEM COMPRISING SUCH A BONE PLATE**

(30) Priority: 29.12.2015 US 201562272435 P; 15.06.2016 US 201615183361
(62) Divisional of application: 16175701.8
(71) Applicant: Orthohelix Surgical Designs, Inc., Medina, OH 44256 (US)
(72) Inventor: LEITHER, Andrew, AKRON, 44333 (US); PRASAD, Priya, SUNRISE, 33322 (US)
(74) Representative: Lavoix

(57) **Abstract**

This bone plate comprises a proximal surface, a distal surface, and a first thickness defined from the distal surface to the proximal surface; a first screw hole, a second screw hole; and, a recessed portion connecting a first through-hole to a second through-hole, the recessed portion configured to at least partially receive a bone staple.

## Description

The present invention relates to a bone plate and to a bone assembly comprising such a bone plate. More generally, the invention relates to an orthopedic construct comprising an orthopedic plate that provides for improved compression in associated bone, in particular, to achieve fusion. The plate can, in the alternative, include one or more compression screws, one or more staples, or both. The construct is capable of inducing compression into the associated bone, and in an alternative embodiment, the plate has a translating portion which is associated with at least either a screw (i.e., compression screw) or one leg of a bone staple.

### BACKGROUND OF THE INVENTION

Orthopedic plating has been practiced for some time to repair osteotomy sites, bone fractures, and other sites where bones need to be held in close proximity to facilitate bone healing, in particular bone fusion. Typically a bone plate is affixed by means of screws to two or more bone portions so as to immobilize the portions relative to each other and thereby facilitate formation of a bone bridge or callus between the two portions. In some cases a bone plate and screws are oriented and tightened to provide a fixed amount of initial compressive force between the bone portions by drawing the bone portions towards each other. In some cases, a bone screw will couple with a compression slot in a bone plate and provide initial compressive force between the bone portions by drawing the portions towards each other by a fixed distance. Generally speaking, with these approaches, the amount of compression between the bone portions diminishes over time due to one or more of loosening of the screws and plates, remodeling of the bones, applied mechanical forces, or other reasons. Since bone is a living organism, it reacts to the load applied in ways that are different than non-living materials, such as metal, plasterboard or wood. It is known that the amount of compression applied to the bone by fasteners can change or reduce over time, due to necrosis or erosion of bone.

Another limitation of using plates for fixation of bone portions is that while the portions in contact with the plate may be immobilized relative to each other the portions of the bone furthest away from the plate may not be sufficiently immobilized relative to each other due to flexing of the plate, or bone, or both. This is a particular problem in load bearing situations. For example, a plate applied to the dorsal surface of a metatarsal may immobilize the bone portions adjacent to the plate yet the bone portions on the plantar side of the metatarsal may suffer significant movement relative to each other during application of force to the metatarsal by, for example, standing on the foot.

In another approach, staples can be used to provide a fixed amount of initial compressive force between two or more bone portions by drawing the portions towards each other at the crown of the staple. In some staples the legs of the staples may be angled such that the ends of the legs are closer to each other than the separation of the legs at the crown. Such staples may be initially placed in an elastically deformed state and thereby also apply a compressive force that continues over time at the ends of the staple legs, for a much longer time than the initial compressive force described above for plates, particularly if the staple is comprised of a superelastic alloy, for example, Nitinol. The force so applied is below the surface of the bone portions. However, a disadvantage of using staples for fixation of bone portions is that the crown of the staple is far less resistant to bending than a plate. In load bearing applications the use of staples is insufficient to immobilize the bone portions over time.

It is desirable to have an orthopedic plating construct that can immobilize bone portions in load bearing applications where the portions are initially compressed into close proximity with each other, the construct prevents motion between the portions due to applied loads, and the construct applies compressive force to immobilize the portions over the healing time.

It is desirable to have an orthopedic plating construct that can achieve joint compression on the side of the bone farthest away from the plating surface (i.e., plantar compression with dorsal plating).

It is desirable to have an orthopedic plating construct that can improve upon the strength and stiffness of staple-only constructs and improves compression in the presence of plate-only constructs.

### SUMMARY OF THE INVENTION

The present invention provides a system for combining a staple and plate, and has utility particularly in midfoot plating where access and plating is done dorsally (compression side of the joint) and there is concern with plantar (tension side of the joint) gapping of the joint.

One object of the invention is a bone plate as defined in claim 1. Additional advantageous features of this bone plate are specified in claims 2 to 11. Thus, in one embodiment, the plate of the present invention has a proximal surface, a distal surface, and a first thickness defined from the distal surface to the proximal surface; a first screw hole, a second screw hole; and, a recessed portion connecting a first through-hole to a second through-hole, the recessed portion configured to at least partially receive a bone staple. Further this bone plate (e.g. in particular as set forth in claim 1), wherein the recessed portion is recessed from the proximal surface of the bone plate. The bone plate has a recessed portion disposed between the proximal and distal surface of the bone plate. The recessed portion may also be positioned between the first and second screw hole. Further, the bone plate may have a second thickness defined from the distal surface of the bone plate to the proximal surface of the recessed portion and in embodiments, the second thickness is less than the first thickness. Bone plates may further have at least one internally threaded through-hole. Further, the first through-hole and the second through-hole may be configured to receive at least a portion of a first staple leg and a second staple leg, respectively and the recessed portion is configured to at least partially receive a staple bridge. The bone plates may have at least one threaded screw hole and at least one of the screw holes may be a compression screw hole. In some embodiments, the first screw hole is a compression screw hole which has a lateral shoulder which forms an edge that sloped toward the proximal surface of the plate.

Methods of conducting surgery are also disclosed including providing a bone plate including a distal surface and a proximal surface, a first screw hole, a second screw hole and a recessed portion recessed from the proximal surface of the plate, the recessed portion inter-connecting a first through-hole to a second through-hole, exposing a bone surface; positioning the bone plate on the bone surface; placing at least a first of a compression, non-locking or locking screw at least partially through the first screw hole of the bone plate; placing a staple at least partially though the bone plate, a first staple leg at least partially received within the first through hole, a second staple leg at least partially received within the second through-hole and a staple bridge positioned adjacent the recessed portion; and, placing at least a second compression, non-locking or locking screw at least partially through the second screw hole of the bone plate.

Another object of the invention is a bone plate and bone staple comprising the bone staple having a bridge which spans a first leg and a second leg and the plate having a first surface and a second surface including a first screw hole, a second screw hole, and a recessed portion configured to receive a portion of the staple bridge, wherein the recessed portion interconnects a first through-hole and a second through-hole sized to receive the first leg and the second leg respectively therethrough. Thus, an embodiment of the invention includes a bone plate having a first surface and a second surface including a first screw hole, a second screw hole, and a recessed portion configured to receive a portion of the staple bridge, wherein the recessed portion interconnects a first through-hole and a second through-hole sized to receive the first leg and the second leg respectively therethrough and bone staple having a bridge which spans a first leg and a second leg.

Another object of the invention is a bone system as defined in claim 12. Additional features of this bone system are specified in claims 13 to 15. Thus, an embodiment of the invention includes a bone plate assembly wherein a plate has a first surface and a second surface; a bone staple has a staple bridge, and first leg and a second leg extending from the staple bridge at an inside angle of from 45° to 135° from the staple bridge; and, wherein the first plate surface has a recess that at least partially receives the staple bridge and including a first through hole that accommodates the staple first leg and a second through hole that accommodates the staple second leg.

Further, the recess may define an intermediate surface facing the direction of the first plate surface and the bridge second surface contacts the intermediate surface in use. Optionally, the plate may have a compression slot having an edge defining a shoulder that slopes toward the plate second surface.

The plate may have at least one threaded, locking screw hole and in embodiments, two threaded screw holes that are separate and distinct from the first and second through-holes. The first through-hole may be partially threaded and the second through-hole may have a compression ramp with an internal shoulder that slopes downward in a direction away from the first through hole.

Screws may be used with the bone plate assembly and can be non-locking, locking, compression screws and combinations thereof. In one particular embodiment, one screw may be a compression screw and another may be a locking screw.

Further, bone plate assemblies may include drill means capable of drilling a tapered hole.

Methods for conducting surgery are also described including the steps of providing a bone plate including a first surface and a second surface, a slot extending therethrough from the first surface to the second surface, the slot further including a first through-hole that accommodates the staple first leg and a second through-hole that accommodates the staple second leg, and a first screw hole and a second screw hole; exposing a bone surface, placing a staple therethrough the bone surface, the staple comprising a bridge having a first surface and a second surface, and first leg and a second leg extending from the bridge at an angle of from 45° to 135° from the bridge; placing the bone plate slot around the staple, on the exposed bone surface wherein the first through-hole is disposed around the first staple leg, and the second through-hole is disposed around the second staple leg; wherein after the bone plate is placed on the bone surface, and placing at least one of a compression, non-locking or locking screw therethrough the screw hole of the bone plate.

In a further aspect of the present disclosure, a bone plate is disclosed having a long axis and first surface and a second surface, the bone plate first surface having a slot that accommodates a staple bridge, the slot including a first through hole that accommodates the staple first leg and a second through hole that accommodates the staple second leg and the plate including a translation member having a threaded locking screw hole wherein the translation member is configured to translate along the long axis distal to the slot.

In yet another embodiment of the present disclosure, a bone plate assembly includes a plate fixed member having a first longitudinal axis, a first screw hole disposed near a plate fixed member first end portion, a first surface and a second, opposing bone facing surface, the plate first surface having a recess near a plate fixed member distal end that accommodates a staple bridge, the plate further including a first through-hole that accommodates a staple first leg and a second through-hole that accommodates a staple second leg, and a plate translation member having a second longitudinal axis and a second screw hole disposed near a translation member second end portion, wherein the plate translation member second end portion is movably coupled to the plate fixed member first end portion. The plate translation member may permit or enable the second screw hole to be translated across the second longitudinal axis. The first and second longitudinal axis may be parallel or the same axis. Further, the translation member can be moveable along at least one of the first longitudinal axis or the second longitudinal axis, relative to the fixed member.

The bone plate assembly may have first and second screw holes disposed along the first longitudinal axis. Further, the second screw hole may be a threaded locking screw hole. In some embodiments, the first and second through-holes include internal threads and the second through-hole can have a compression ramp having an internal shoulder that slopes downward in a direction away from the first through-hole.

The bone plate assembly may include a recess defining an intermediate surface, the surface facing the direction of the first plate surface. The bone plate assembly may also include a bone staple and the bone staple has a second bridge surface which contacts the plate intermediate surface when in use. Alternatively, the plate includes a compression slot.

Methods for conducting surgeries are described herein including providing a plate including fixed member having a first longitudinal axis, a first screw hole disposed near a plate fixed member first end portion, a first surface and a second, opposing bone facing surface, the plate first surface having a recess near a plate fixed member distal end that accommodates a staple bridge, the plate further including a first through-hole that accommodates a staple first leg and a second through-hole that accommodates a staple second leg, and a plate translation member having a second longitudinal axis and a second screw hole disposed near a translation member second end portion, wherein the plate translation member second end portion is movably coupled to the plate fixed member first end portion; placing the plate on a bone surface; positioning the staple bridge thereon the recess, wherein the first and second staple legs are disposed therethrough the first and second through-holes, respectively; positioning at least one of a locking, non-locking and compression screw therethrough the plate and bone surface; and closing the exposed bone surface.

Other orthopedic plate systems described herein include a locking screw, a compression screw, and a staple, and an orthopedic plate having a translation portion including a translation portion screw hole and a locking portion including a locking portion screw hole, and at least one of a slot or recessed portion disposed between the translating portion screw hole and the locking portion screw hole. The slot may extend from a first distal surface of the plate to a second proximal surface of the plate. In certain embodiments, the slot is shaped to receive at least a portion of the staple. Alternatively, the recessed portion is disposed between a first and a second through-hole. The recessed portion may also be configured to at least partially receive a portion of the staple. Further, the translation portion is moveable with respect to the locking portion. The first through-hole may be shaped to receive a first staple leg and the second through-hole may be shaped to receive a second staple leg. The locking screw may be a fixed angle locking screw.

Other methods are surgery described herein include a method of surgery to achieve active compression in at a bone interface having at least a bone and comprising the steps of: exposing the bone interface; placing an orthopedic plate on the bone and fastening it to the bone with a first fastener and a second fastener, and optionally a third fastener, where either the second or the third fastener is a compression fastener and the orthopedic plate having a translation portion having a translation portion fastener hole and a locking portion having a locking portion fastener hole, wherein a first fastener is a locking fastener and installing the first fastener in the locking portion fastener hole to form a locked relationship and installing the second fastener in the translation portion fastener hole and installing the compression fastener to drive active compression in the translation portion. According to optional features of this method:
- the second compression screw cooperates with the translation portion screw hole and a compression screw drives active compression in the translation portion;
- the locking screw cooperates with the locking portion screw hole to form a locked relationship;
- the bone interface is the interface between a first bone segment and a second bone segment and the active compression is driven at the bone interface.

Other orthopedic plate described herein has a translation portion and a locking portion, wherein a compression fastening element drives active compression in the translation portion. In some embodiments, the translation portion may include a fastener hole and/or the translation portion fastener hole may be a locking screw hole and/or the compression fastening element may be a staple and/or the staple may comprise an elastic element and/or the orthopedic plate may have a medial line plane and the locking portion and the translation portion may be aligned and may translate relative to each other in the direction of the medial plane and/or one of the locking portion and the translation portion may have a female element and the other of the locking portion and the translation portion may have a male element which mates with the male element to allow the translation of the translation portion relative to the locking portion and/or the female element may be a recess and/or the recess may be V-shaped and/or the male element may be V-shaped and the male element may dovetail with the female element.

### BRIEF DESCRIPTION OF THE DRAWINGS

Figure 1 is a top perspective view of the unassembled active compression plate, staple and fasteners in accordance with one embodiment of the present invention;
Figure 2 is a side view of the staple of Figure 1;
Figure 3 is a side perspective view of the staple of Figure 1;
Figure 4 is a cross-section of the active compression plate of Figure 1 taken along line A-A;
Figure 5 is a top view of the active compression plate and staple and locking screw assembly of Figure 1;
Figure 6 is a side view of the active compression plate and staple and locking screw assembly of Figure 1;
Figure 7 is a side perspective view of the active compression plate of Figure 1;
Figure 8 a side perspective view of the active compression plate assembly of Figure 1;
Figure 9 is top view of another embodiment of the active compression plate in accordance with the invention;
Figure 10 is a cross-section of the active compression plate of Figure 9 taken along line B-B;
Figure 11 is a top view of the active compression plate of Figure 9 with assembled locking screws and a compression screw;
Figure 12 is a top view of the active compression plate of Figure 9 with assembled locking screws and a staple;
Figure 13 is a side perspective view of the active compression plate assembly of Figure 12;
Figure 14 is a side perspective view of the active compression plate assembly of Figure 11;
Figure 15 is top view of yet another embodiment of the active compression plate including a translating section in accordance with the invention;
Figure 16 is a cross-section of the active compression plate of Figure 15 taken along line C-C;
Figure 17 is a side view of the active compression plate of Figure 15;
Figure 18 is a bottom view of the active compression plate of Figure 15;
Figure 19 is a top side perspective view of the active compression plate of Figure 15;
Figure 20 is an exploded view of the active compression plate of Figure 15;
Figure 21 is a top view of the active compression plate of Figure 15 with assembled locking screws and with a staple;
Figure 22 is a cross-section of the active compression plate assembly of Figure 21 taken along line D-D;
Figure 23 is a side view of the active compression plate assembly of Figure 21;
Figure 24 is an exploded bottom perspective view of the active compression plate of Figure 15;
Figure 25 is a bottom view of the active compression plate of Figure 15;
Figure 26 is a detail of the telescoping mechanism for the translating and locking portions of the active compression plate of Figure 15;
Figure 27 is a cross-section of the active compression plate of Figure 15 taken at line E-E;
Figure 28 is a top view of an alternate embodiment of the active compression plate of the present invention including an alternative locking and translating profile;
Figure 29 is a bottom view of the active compression plate of Figure 28 illustrating the translating portion in a first position;
Figure 30 is a bottom view of the active compression plate of Figure 28 illustrating the translating portion in a second position;
Figure 31 is a cross-section of the translating active compression plate of Figure 28 taken at line F-F;
Figure 32 is an exploded view of the translating active compression plate of Figure 28;
Figure 33 is a top view of another embodiment of the active compression plate of the present invention;
Figure 34 is a side perspective view of the active compression plate of Figure 33;
Figure 35 is a top view of the active compression plate assembly using the active compression plate of Figure 33 and including first and second locking screws and a staple;
Figure 36 is a cross section of the active compression plate assembly of Figure 33 taken at line G-G;
Figure 37 is a side perspective view of the active compression plate of Figure 36;
Figure 38 is a side view of the active compression plate of Figure 33 taken along lines H-H;
Figure 39 is a top view of yet another embodiment of the present invention including a compound active compression plate and in which each plate segment includes first and second locking screws and a staple;
Figure 40 is a top view of the compound active compression plate of Figure 39;
Figure 41 is a side view of the compound active compression plate of Figure 39;
Figure 42 is an anterior/lateral view of the skeleton of a foot including two active compression plate assemblies as shown in Figure 35-37; and,
Figure 43 is a dorsal view of the skeleton of the foot and active compression plate assemblies of Figure 39.

### DETAILED DESCRIPTION OF THE INVENTION

### Embodiment 1 - full thickness slot

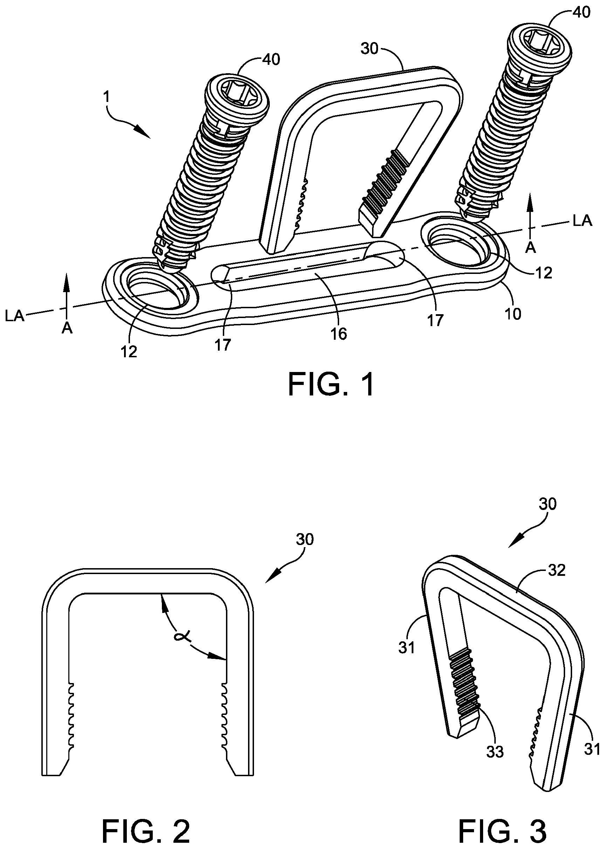

Figure 1 illustrates active compression plate system 1 in an unassembled configuration, comprised of bone plate 10, bone staple 30, and one or more fasteners 40 in accordance with a first embodiment of the invention. In one example of use plate 10 is placed against a patient's bone, one fastener 40 is inserted through fastener hole 12 of plate 10 and affixed to the bone, one staple 30 is inserted through plate slot 16 and into the patents bone, and a second fastener 40 is inserted through fastener hole 12 in plate 10 and affixed to the bone. It should be noted that the term "slot", refers to herein specifically as a full thickness recess or full thickness opening which extends from a first proximal plate surface to a second distal plate surface. With reference to Figures 1 to 8, active compression plate system 1 will be further described below.

Bone plate 10 may have a profile looking down from the top of a modified rectangle, with equivalent opposing parallel long sides 21 defining straight lateral edges 22, and with rounded short ends 23 having circular lateral edges 24. Bone plate 10 is comprised of a proximal surface 13 and a distal surface 14 having a first thickness 15 therebetween, at least one slotted portion 16, one or more through holes 17 and one or more fastener holes 12. As illustrated in FIGS. 1-8, slotted portion or slot extends all the way through the plate, from a first surface to a second surface of the plate. Slotted portion 16, through holes 17 and fastener holes 12 may be generally aligned along plate longitudinal axis LA. In one embodiment plate is comprised of one slot, two through holes and two fastener holes. Fastener holes 12 may be smooth walled, threaded, for example for use with threaded fasteners, or formed as compression slots having an angled shoulder that cooperates with a screw to apply a transverse motion to the plate as the screw is tightened against a bone. Optionally bone plate 10 may be comprised of one, two, three, four, five, six, or more fastener holes (not shown) for use with various fasteners as is known in the art.

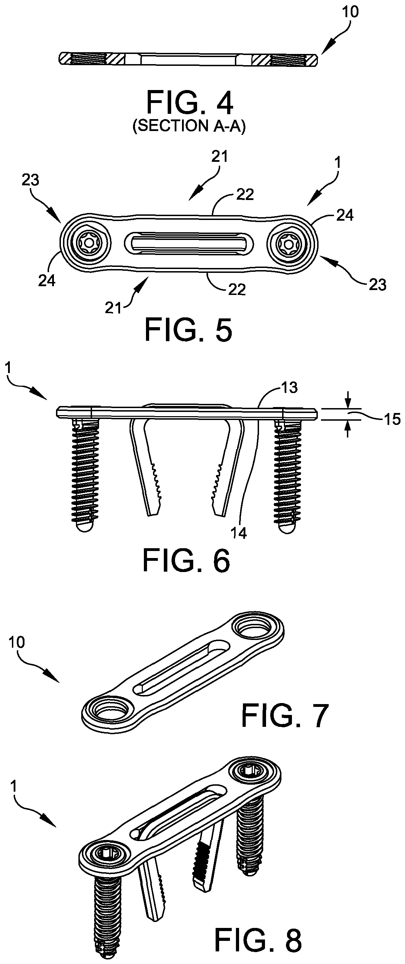

In use slot 16 accommodates one or more staple crowns as will be described below. Slot may extend over the full thickness of plate as shown in Figs. 1 and 7 and have the characteristics of an opened bottom slot. Slot terminates at through openings 17 that accommodate the legs of the staple when it is assembled with the plate. Slot 16 is intermediate or disposed between the screw holes 12. Slot is sized to accommodate staple bridge. It should be understood that the term staple "bridge" as used herein defines the portion of the staple that connects or is disposed between the two staple legs. Slot dimensions are larger than staple bridge dimensions such that bridge can pass through slot in a sliding fit or a less snug fit. In an alternative embodiment slot is sized to interact with staple bridge as an interference fit.

It should be noted that in certain embodiments described herein, the slot or slotted portion, extending the full thickness of the plate may be interchanged with a recess or recessed portion, having a partial thickness of the plate.

Bone plate 10 can be curved along longitudinal axis LA or normal to axis LA, or both. In one embodiment plate 10 is curved or contoured to mimic the typical shape of bone surfaces at the intended implantation location. In another embodiment plate 10 is curved or contoured to mimic the shape of the specific bone surface onto which the plate will be affixed.

Bone plate 10 may comprise of titanium, stainless steel, Nitinol, or other metals; Poly Ether Ether Ketone (PEEK) or other polymers; alumina or other ceramics, bioabsorbable materials including metal, ceramic, or polymer, in whole or in part, or combinations thereof, or other materials.

Bone staple 30 is comprised of two or more legs 31 joined by bridge 32. An included angle α between leg 31 and bridge 32 (see Figure 2) falls between 45° and 135. During insertion of staple 30 into the patient's bone angle α is held at 90° +/- 10°, and preferably +/-5°, and more preferably +/- 3°) in order to facilitate insertion of the staple into the bone, and once implanted tends to return to a pre-programmed second configuration as shown in Figure 3 in which the legs angle inward toward each other by 5-20° from the insertion position. Legs and bridge may be generally straight (as illustrated) or curved. Staple 30 may be comprised of barbs or other gripping structure 33, which, in certain embodiments, may be disposed only on the staple legs to facilitate secure locking of staple 30 to a patient's bone.

Bone staple 30 may be comprised of a wire like structure that has been formed into a shape similar to that of the English language letter U.

Bone staple 30 may comprise of titanium, stainless steel, Nitinol, or other metals; polyetheretherketone (PEEK) or other polymers; alumina or other ceramics, bioabsorbable materials including metal, ceramic, or polymer, in whole or in part, or other materials. Superelastic Nitinol is a preferred material of construction for staples used in the active compression plate system 1 due to the large amount of elastic strain (up to 9%) that superelastic nitinol can reversibly sustain.

Fastener 40 may be comprised of a non-locking bone screw, a locking bone screw, a compression bone screw, a peg, or other fasteners. In general, bone screws are preferred. When more than one fastener is used in active compression plate system 1 the fasteners used can be any combination of non-locking bone screws, locking bone screws, compression bone screws, pegs, or other fasteners.

Fastener 40 may be comprised of titanium, stainless steel, Nitinol, or other metals; Poly Ether Ether Ketone (PEEK) or other polymers; alumina or other ceramics, bioabsorbable materials including metal, ceramic, or polymer, in whole or in part, or other materials.

In an exemplar method of use of active compression plate system 1, a patient is prepared for surgery and surgical access to one or more bones is achieved. Access may be accomplished by open surgery, small incision surgery, or percutaneously.

The surgical procedure chosen, planned, or performed will be for repair of bone fracture, repair of osteotomy, bone fusion, or other procedures in which two or more bones or bone portions must be held in close proximity to each other to facilitate bone healing, and in particular to facilitate bone fusion.

The patient's bone or bone portions are exposed and reduced to the desired positions.

Bone staple 30 is placed into the bone or bone portions and the staple is allowed to hold the bone or bone portions at the desired positions relative to each other.

Slot 16 of plate 10 is passed over bridge of staple 30 and plate 10 is brought into contact with the patient's bone.

Optionally a drill guide is inserted into at least one fastener hole 12 and a hole is drilled into the patient's bone.

Plate 10 is affixed to at least one bone or bone portion by means of inserting at least one fastener 40 such as a bone screw into fastener hole 12 and into the drilled hole (if any) then advancing the fastener into the patient's bone. In a preferred embodiment plate 10 is affixed to at least two bones or bone portions by means of at least two bone screws.

In one particular embodiment, a method of conducting a surgery includes providing a bone plate including a first surface and a second surface, a slot extending therethrough from the first surface to the second surface, the slot further including a first through-hole that accommodates the staple first leg and a second through-hole that accommodates the staple second leg, and a first screw hole and a second screw hole; exposing a bone surface; placing a staple therethrough the bone surface, the staple comprising a bridge having a first surface and a second surface, and first leg and a second leg extending from the bridge at an angle of from 45° to 135 ° from the bridge; placing the bone plate slot around the staple, on the exposed bone surface wherein the first through-hole is disposed around the first staple leg, and the second through-hole is disposed around the second staple leg; wherein after the bone plate is placed on the bone surface, and placing at least one of a compression, non-locking or locking screw therethrough the screw hole of the bone plate.

### Embodiment 2 - compression slot

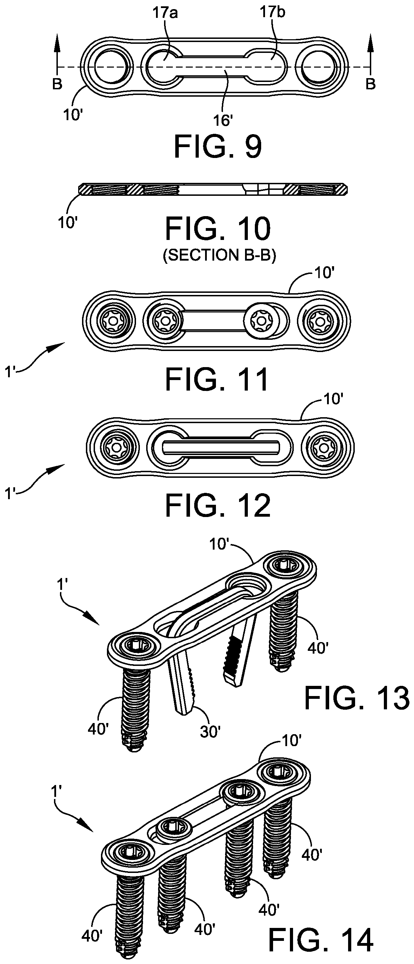

Figures 9 to 14 illustrate active compression plate system second embodiment 1', comprised of bone plate 10', bone staple 30', and one or more fasteners 40'. In this second embodiment a plate slot can be coupled with either a staple or with screws, as further described below.

In active compression plate system second embodiment 1', bone plate 10', bone staple 30', and fasteners 40' have a similar structure and are made of the same materials as bone plate 10, bone staple 30, and fasteners 40 of active compression plate system 1, discussed above, except as noted below.

In active compression plate system second embodiment 1', slotted portion 16' of plate 10' terminates at one end in a threaded through-hole 17a which can accommodate fastener 40', in one embodiment a threaded locking screw and at the other end terminates in a second through-hole which is a ramped compression slot 17b, accommodating fastener 40', in one embodiment a non-locking screw, to cause active compression by means of bone screws instead of by means of a bone staple. In other embodiments, as illustrated in Figure 13, the first through-hole 17a and second through-hole 17b are shaped to at least partially receive a staple 30', and in particular a first staple leg and a second staple leg.

In an exemplar method of use of active compression plate system 1', a patient is prepared for surgery and surgical access to one or more bones is achieved. Access may be accomplished by open surgery, small incision surgery, or percutaneously.

The surgical procedure chosen, planned, or performed will be for repair of bone fracture, repair of osteotomy, bone fusion, or other procedures in which two or more bones or bone portions must be held in close proximity to each other to facilitate bone healing, and in particular to facilitate bone fusion.

The patient's bone or bone portions are exposed and reduced to the desired positions.

The surgeon considers whether the appropriate form of treatment will be to insert staple 30' into the bone or bone portions, followed by use of plate 10', or to use fasteners 40' as an alternative to staple 30'.

In the case where the surgeon decides to insert staple 30' into the bone or bone portions the method of use from this decision onwards adheres to the method described in connection with active compression plate system 1.

In the case where the surgeon decides to insert fasteners 40' into the bone or bone portions, optionally a drill guide is inserted into at least fastener hole 17a and a hole is drilled into the patient's bone.

Plate 10' is positioned such that slot 16' straddles a gap between two adjacent bones or bone portions.

Plate 10' is affixed to at least one bone or bone portion by means of inserting at least one fastener 40' such as a bone screw into through hole 17a, into the drilled hole (if any) and then advancing the fastener into the patient's bone.

Plate 10' is further affixed to at least one bone or a second bone portion by means of inserting at least one fastener 40' such as a bone screw into ramped compression slot 17b then advancing the fastener into the patient's bone thereby causing bone portions to move towards each other and closing any gap between the portions.

Optionally a drill guide is inserted into at least one fastener hole 12' and a hole is drilled into the patient's bone.

Plate 10' is further affixed to at least one bone or bone portion by means of inserting at least one additional fastener 40' such as a bone screw into fastener hole 12' and into the drilled hole (if any) then advancing the fastener into the patient's bone. In a preferred embodiment plate 10' is affixed to at least two bones or bone portions by means of at least three, and preferably four, bone screws.

### Embodiment 5 - bent plate, partial thickness recess

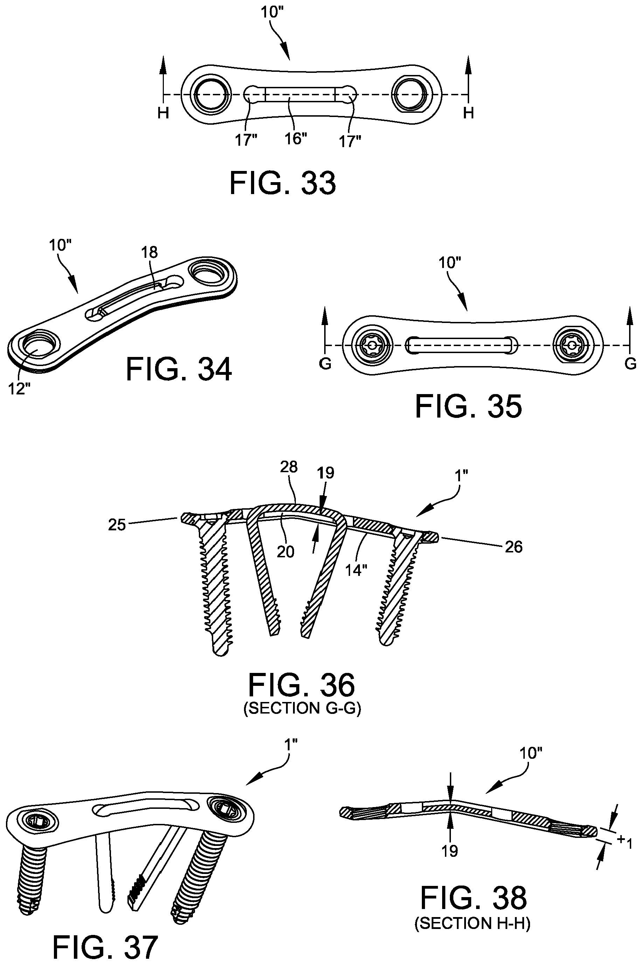

Figures 33 to 38 and 42 illustrate active compression plate system fifth embodiment 1", comprised of bone plate 10", bone staple 30", and one or more fasteners 40". In this fifth embodiment plate 10" has a narrowed central width and is bent out of plane, as further described below.

In active compression plate system fifth embodiment 1", bone plate 10", bone staple 30", and fasteners 40" have the same structure and are made of the same materials as bone plate 10, bone staple 30, and fasteners 40 of active compression plate system 1, discussed above, except as noted below.

Bone plate 10" is comprised of a taper inward or centrally from the ends of the plate as it progresses toward the transverse middle portion of the plate as is apparent from Fig 35.

Bone plate 10" is also comprised of an angled or bent portion in the intermediate portion nearer to one end than the other, and in one embodiment is designed for a dorsal fusion in the mid-foot. With reference to Figure 36, one end of plate 10" aligns longitudinally with first medial axis 25 and the other end of plate 10" aligns longitudinally with second medial axis 26. Medial axes 25 and 26 intersect at angled or bent portion 28 such that the included angle between axes 25 and 26 is acute at the distal surface 14" of plate 10".

Bone plate 10" is also comprised of recess 16" that may extend over a partial thickness of the plate as shown in Figs. 34 and 36, having a proximally facing surface 18, a second thickness 19, and web 20 spanning the portion of the plate distal to the recess. In particular, recess 16" is recessed from proximal surface of the plate as can be best seen in Figures 36 and 38. Further, recess, is disposed between first and second screw holes. Recessed surface may also be angled similarly as described above with regards to medial axes 25 and 26. It should be noted that second thickness 19 is less than full thickness of the bone plate t1.

It should be noted that in embodiments described herein, the slot or slotted portion, extending the full thickness of the plate may be interchanged with a recess or recessed portion, having a partial thickness of the plate.

In an exemplar method of use of active compression plate system 1", a patient is prepared for surgery and surgical access to one or more bones is achieved. Access may be accomplished by open surgery, small incision surgery, or percutaneously.

The surgical procedure chosen, planned, or performed will be for repair of bone fracture, repair of osteotomy, bone fusion, or other procedures in which two or more bones or bone portions must be held in close proximity to each other to facilitate bone healing, and in particular to facilitate bone fusion.

The patient's bone or bone portions are exposed and reduced to the desired positions.

Plate 10" is positioned such that recess 16" straddles a gap between two adjacent bones or bone portions.

Optionally a drill guide is inserted into at least one fastener hole 12" and a hole is drilled into the patient's bone

Plate 10" is affixed to at least one bone or bone portion by means of inserting at least one fastener 40" such as a bone screw into fastener hole 12" and into the drilled hole (if any) then advancing the fastener into the patient's bone.

Bone staple 30" is placed through recess 16", through holes 17", and into the bone or bone portions and the staple is allowed to hold the bone or bone portions at the desired positions relative to each other.

Plate 10" is affixed to at least one bone or bone portion by means of inserting a second fastener 40" such as a bone screw into hole 12", into a drilled hole (if any) and then advancing the fastener into the patient's bone.

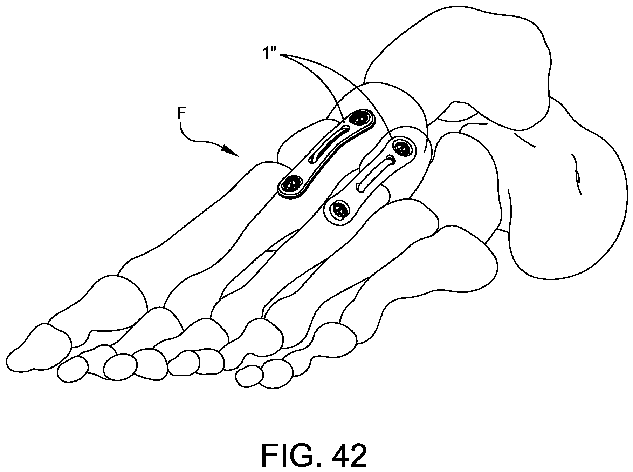

Active compression plate system 1" implanted on a patient's mid-foot F in a dorsal fusion procedure is represented in Fig. 42.

In another particular method of surgery, a bone plate 10" is provided including a distal surface and a proximal surface, a first screw hole, a second screw hole and a recessed portion 16" recessed from the proximal surface of the plate, the recessed portion 16" inter-connecting a first through-hole 17" to a second through-hole 17", exposing a bone surface; and positioning the bone plate 10" on the bone surface. Next, at least a first compression, non-locking or locking screw is at least partially inserted through the first screw hole of the bone plate. Then, the staple is at least partially received though the bone plate wherein a first staple leg at least partially received within the first through hole, a second staple leg at least partially received within the second through-hole and a staple bridge positioned adjacent the recessed portion. In particular, the staple bridge includes a first proximal surface (not labeled) and second distal surface (not labeled) and the second distal surface of the staple bridge is positioned adjacent the recessed portion proximal surface 18. Lastly, a second compression, non-locking or locking screw is positioned at least partially through the second screw hole of the bone plate.

### Embodiment 6 - compound plate

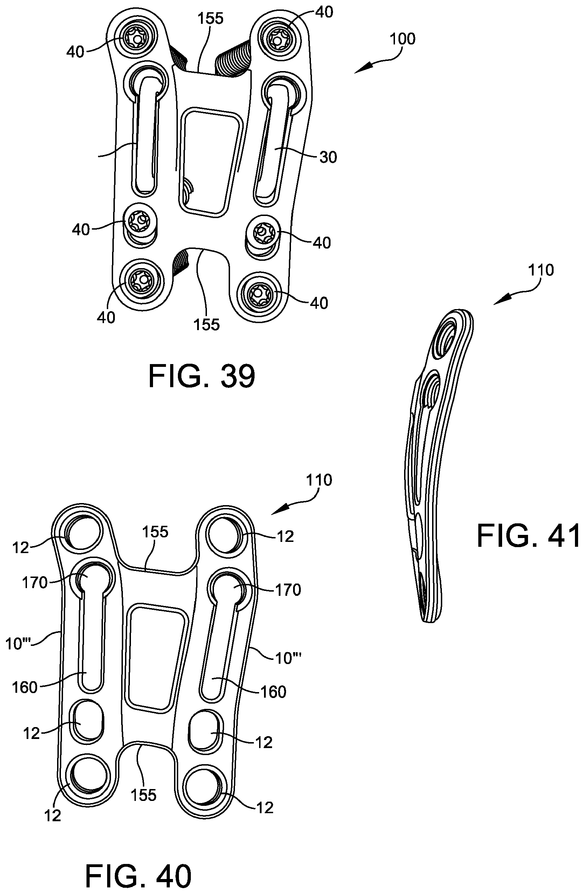

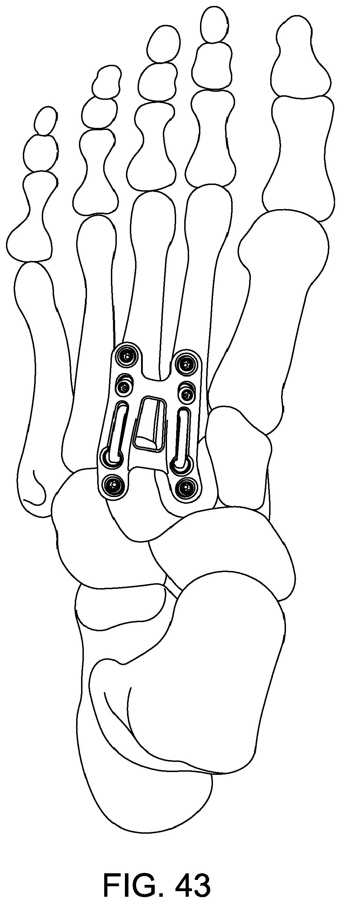

Figures 39 to 41 and 43 illustrate active compression plate system sixth embodiment 100, comprised of staples 30, fasteners 40, and two or more of active compression plate systems 1, 1', 1" joined together by means of linking sections 155. It is contemplated that in other embodiments 3, 4, or 5 of systems 1, 1', 1" can be joined together by linking sections 155. In compounded plate system 100 compound bone plate 110 is implanted to promote fusion between multiple adjacent bones and/or bone portions. In one embodiment, system 100 is implanted to promote dorsal midfoot bone fusion as shown in Fig. 43. Other embodiments are particularly well suited for midfoot procedures involving adjacent bones of the foot (i.e., for adjacent rays).

In compound plate 110, individual plates 10"' are linked together such that the longitudinal axes of the plates are not parallel, and so, while the recessed portions 16 and holes 12 are at least roughly aligned along the long axes of the individual plates 10"', the rows of holes in adjacent plates 10‴ are not parallel. In another embodiment the rows of holes in adjacent plates 10‴ can be parallel or disposed along longitudinal axes which may be parallel to one another.

In compound plate 110 linking sections 155 are comprised of multiple elongate tape-shaped sections of plate that are oriented generally transverse to plate 10‴ longitudinal axes. In one embodiment, linking sections 155 have similar structure to bone plates 10, 10', 10" and are made of the same materials as bone plates 10, 10', 10". In alternate embodiments, linking sections may be comprised of a biodegradable material which can preferentially degrade the linking sections 155, leaving the individual bone plates intact. In yet alternate embodiments, the compound plate may comprise degradable materials wherein the linking sections or the bone plates may have different degradation rates including mass loss and strength loss, each of which can be tailored or designed depending on the patient and surgical application.

In compounded plate system 100, bone plate 110 is comprised of two or more of bone plates 10"'. Bone plates 10‴ have the same structure and are made of the same materials as bone plates 10, 10', 10" of active compression plate systems 1, 1', 1", discussed above, except as noted below. It is understood that bone plate 10‴ may be comprised of an angled or bent portion in the intermediate portion, similar to bone plate 10". Bone staples 30, fasteners 40, and fastener holes 12 have the same structure and are made of the same materials as bone staples 30, 30', 30", fasteners 40, 40', 40", and fastener holes 12, 12', 12" of active compression plate systems 1, 1', 1", discussed above, except as noted below. Recessed portions 160 and through-holes 170 have the same structure and are made of the same materials as recessed/slotted portions 16, 16', 16" and through-holes 17, 17", 17a, 17b of active compression plate systems 1, 1', 1", discussed above, except as noted below. It is understood that plates 10‴ can be comprised of full thickness slots or partial thickness recesses.

Plate 10‴, as illustrated in Figs. 39 to 41 and 43, varies somewhat from the illustrated structures of plates 10, 10' and 10". Specifically, three fastener holes 12 are provided in the plate, and the central hole 12 is configured as a compression slot. Additionally, recess 160 has through hole 170 configured as a threaded hole similar to hole 17a

In an exemplar method of use of active compression plate system 100, a patient is prepared for surgery and surgical access to one or more bones is achieved. Access may be accomplished by open surgery, small incision surgery, or percutaneously.

The surgical procedure chosen, planned, or performed will be for repair of bone fracture, repair of osteotomy, bone fusion, or other procedures in which two or more bones or bone portions must be held in close proximity to each other to facilitate bone healing, and in particular to facilitate bone fusion.

The patient's bone or bone portions are exposed and reduced to the desired positions.
A. Plate 100 is positioned such that recess 160 of first plate 10"' straddles a gap between two adjacent bones or bone portions AND second plate 10‴ is positioned over adjacent bones and/or bone portions such that they also can be immobilized in the desired positions by the cooperation of first and second bone plates 10‴.
B. Optionally a drill guide is inserted into at least one fastener hole 12 and a hole is drilled into the patient's bone
C. First plate 10‴ is affixed to at least one bone or bone portion by means of inserting at least one fastener 40 such as a bone screw into the fastener hole 12 not adjacent to compression slot 12 and into the drilled hole (if any) then advancing the fastener into the patient's bone.
D. A second fastener 40 such as a bone screw is inserted into hole 12 of first plate 10‴ that is configured as a compression slot, into a drilled hole (if any) and then advancing the fastener into the patient's bone causing the bone portions or bones to be drawn together thereby reducing the gap between bones.
E. Bone staple 30 is placed through recess 160 and through hole 170, then into the bone or bone portions and the staple is allowed to hold the bone or bone portions at the desired positions relative to each other.
F. A third fastener 40 such as a bone screw is inserted into remaining hole 12 of first plate 10"', into a drilled hole (if any) and then advancing the fastener into the patient's bone causing the bone portions or bones to be further locked together in an active compressed state.
G. First plate 10‴ is affixed to at least one bone or bone portion by means of inserting a third fastener 40 such as a bone screw into the remaining hole 12 in first plate 10‴, into a drilled hole (if any) and then advancing the fastener into the patient's bone.

Method steps A through G are repeated for second plate 10‴.

In an alternate method, the staples 30 are placed in the bone followed by placement of the bone plate 100 and fasteners.

### Embodiment 4 - translating screw hole

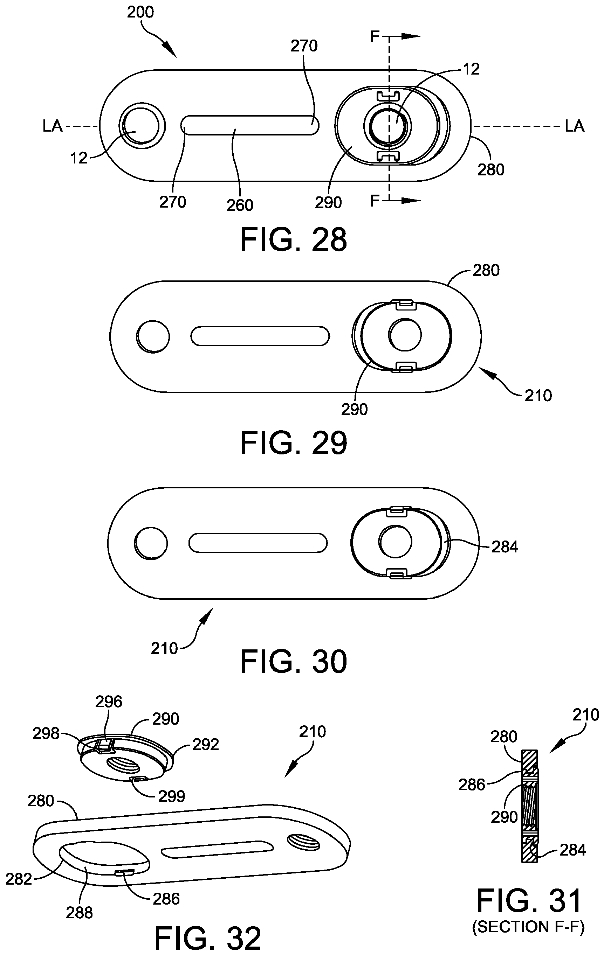

Figures 28 to 32 illustrate active compression plate system fourth embodiment 200, comprised of translating bone plate 210, bone staple 30 (not shown), fasteners 40 (not shown), recessed portion 260, and fastener holes 12. Bone staple 30, fasteners 40, and fastener holes 12 have the same structure and are made of the same materials as bone staples 30, 30', 30", fasteners 40, 40', 40", and fastener holes 12, 12', 12" of active compression plate systems 1, 1', 1", discussed above, except as noted below. Slotted portion 260 is comprised of through holes 270 and has the same structure and is made of the same materials as recessed/slotted portions 16, 16', 16" and through holes 17, 17", 17a, 17b of active compression plate systems 1, 1', 1", discussed above, except as noted below. It is understood that plate 210 can be comprised of full thickness slot or partial thickness recess 260 and that the two may be interchangeable depending on the bone quality, patient anatomy or surgeon preference.

Translating bone plate 210 has longitudinal axis LA and is further comprised of locking portion 280 and translating portion 290. Locking portion 280 and translating portion 290 have the same structure and are made of the same materials as bone plates 10, 10', 10" of active compression plate systems 1, 1', 1", discussed above, except as noted below. It is understood that locking portion 280 may be comprised of an angled or bent portion in the intermediate portion, similar to bone plate 10".

Locking portion 280 is comprised of elongated plate through-hole 282 having plate hole counterbore 284 formed into and below the proximal surface of plate portion 280, and one or more plate hole pocket 286 formed into the inner wall 288 of plate portion 280. Translating portion 290 is comprised of retaining flange 292 formed adjacent to proximal surface of plate portion 290 and one or more snap tabs 296 each having barb 298 at the tab distal end and a clearance relief 299 on the side of the tab opposite the barb.

Locking portion 280 and translating portion 290 are assembled by inserting distal end of translating portion into locking portion plate hole counterbore 284 then into plate through hole 282 so as to reversibly deflect snap tab(s) into clearance relief(s) 299 of plate translating portion. Further insertion causes plate translating portion retaining flange 292 to enter locking portion plate hole counterbore 284 and causes snap tab(s) to deflect outward and into plate hole pocket 286 of plate locking portion. Translating portion 290 is thereby prevented from exiting locking portion 280 by means of retaining flange 292 cooperating with counterbore 284 in combination with barb(s) 298 cooperating with pocket(s) 286. However translating portion 290 is allowed to translate along longitudinal axis LA of locking portion due to designed clearances along longitudinal axis LA between the lengths of plate hole 282, counterbore 284 and flange 292 and between the lengths of barb(s) 298 and pockets 286.

In an exemplar method of use of active compression plate system 200, a patient is prepared for surgery and surgical access to one or more bones is achieved. Access may be accomplished by open surgery, small incision surgery, or percutaneously.

The surgical procedure chosen, planned, or performed will be for repair of bone fracture, repair of osteotomy, bone fusion, or other procedures in which two or more bones or bone portions must be held in close proximity to each other to facilitate bone healing, and in particular to facilitate bone fusion.

The patient's bone or bone portions are exposed and reduced to the desired positions.

Plate 200 is positioned such that recess 260 straddles a gap between two adjacent bones or bone portions.

Optionally a drill guide is inserted into at least one fastener hole 12 of translating portion 290 and a hole is drilled into the patient's bone

Translating portion 290 is loosely affixed to at least one bone or bone portion by means of inserting at least one fastener 40 such as a bone screw into the fastener hole 12 of translating portion 290 and into the drilled hole (if any) then advancing the fastener into the patient's bone.

Any gap between the bone or bone portions immediately underlying plate 200 is reduced and a second fastener 40 such as a bone screw is inserted into hole 12 of locking portion 280, into a drilled hole (if any) and then advanced into the patient's bone causing the bone portions or bones to be locked together in a reduced configuration.

The bone or bone portions are further advanced towards each other to reduce any gap therebetween. This is allowed by system 200 due to the allowable longitudinal motion between translating portion and fixed portion. Fastener 40 on translating portion is immediately advanced to lock translating portion and adjacent locking portion of plate to the bone or bone portions.

Bone staple 30 is placed through recess 260 and through hole(s) 270, then into the bone or bone portions and the staple is allowed to hold the bone or bone portions at the desired positions relative to each other. The staple ends apply active compression forces against the bone at a distance below the bone plate distal surface.

In an alternate method staple 30 can be placed into the bone followed by placement of bone plate 210 and use of the translating aspect of system 200.

### Embodiment 3 - translating plate portion

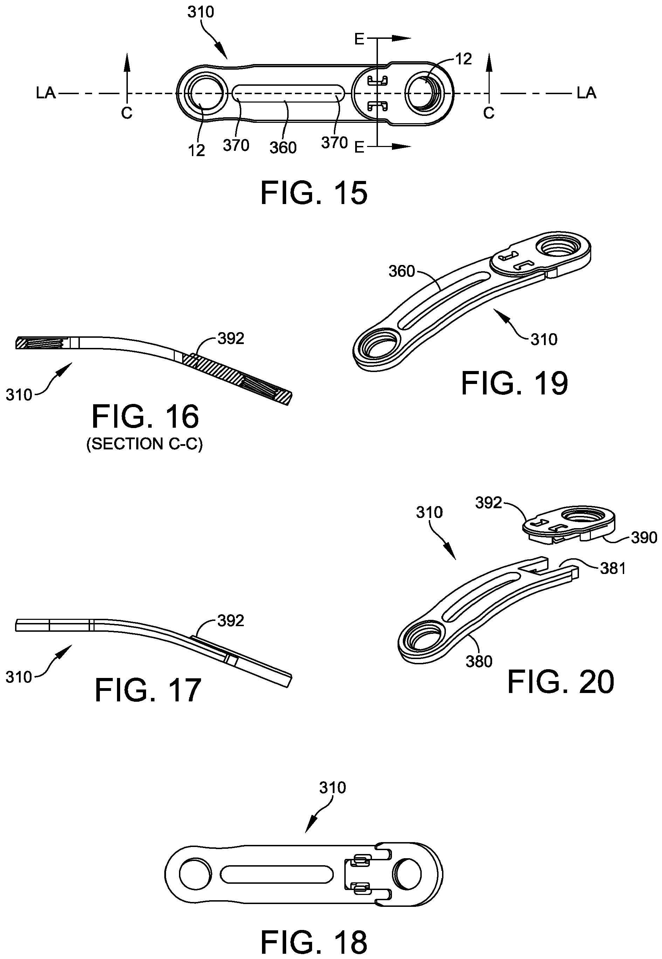

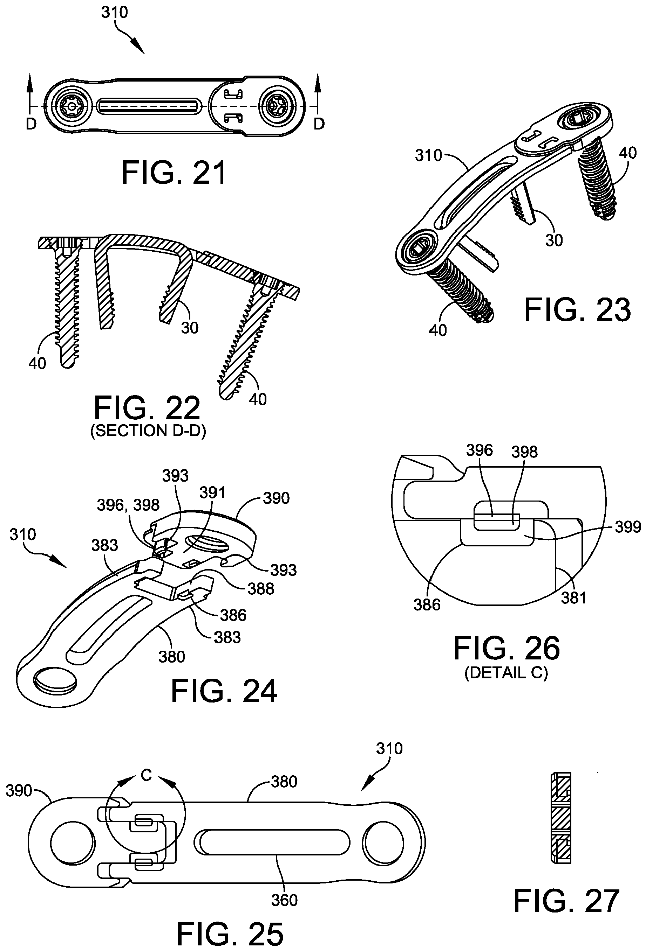

Figures 15 to 27 illustrate active compression plate system third embodiment 300, comprised of translating bone plate 310, bone staple 30, fasteners 40, slotted portion 360, and fastener holes 12. Bone staple 30, fasteners 40, and fastener holes 12 have the same structure and are made of the same materials as bone staples 30, 30', 30", fasteners 40, 40', 40", and fastener holes 12, 12', 12" of active compression plate systems 1, 1', 1", discussed above, except as noted below. Slotted portion 360 is comprised of through-holes 370 and has the same structure and are made of the same materials as recessed/slotted portions 16, 16', 16" and through holes 17, 17", 17a, 17b of active compression plate systems 1, 1', 1", discussed above, except as noted below. It is understood that plate 310 can be comprised of full thickness slot (360) or partial thickness recess 360 (not shown).

Translating bone plate 310 has longitudinal axis LA and is further comprised of locking portion 380 and translating portion 390. Locking portion 380 and translating portion 390 have the same structure and are made of the same materials as bone plates 10, 10', 10" of active compression plate systems 1, 1', 1", discussed above, except as noted below. It is understood that locking portion 380 may be comprised of an angled or bent portion in the intermediate portion, similar to bone plate 10", as illustrated in the figures.

Translating and locking portions cooperate in a telescoping manner. Locking portion 380 is comprised of one or more elongated tab 383, one or more elongated slot 381 and one or more plate pocket(s) 386 formed into the inner wall 388 of plate portion 380. Translating portion 390 is comprised of retaining flange 392, one or more plate elongated slot 393, one or more elongated tab 391, and one or more snap tabs 396 each having barb 398 at the tab distal end and a clearance relief 399 on the side of the tab opposite the barb.

Locking portion 380 and translating portion 390 are assembled by inserting elongated tab 391 of translating portion into elongated slot 381 of locking portion so as to reversibly deflect snap tab(s) into clearance relief(s) 399 of plate translating portion. Further insertion causes plate translating portion retaining flange 392 to contact locking portion plate proximal surface and causes snap tab(s) to deflect outward and into plate hole pocket 386 of plate locking portion. Translating portion 390 is thereby prevented from exiting locking portion 380 by means of retaining flange 392 cooperating with plate 380 proximal surface in combination with barb(s) 398 cooperating with pocket(s) 386. However translating portion 390 is allowed to translate along longitudinal axis LA of locking portion due to designed clearances along longitudinal axis LA between the lengths of plate tabs 381, 391 and plate slots 383, 393 and between the lengths of barb(s) 398 and pockets 386.

In an exemplar method of use of active compression plate system 300, a patient is prepared for surgery and surgical access to one or more bones is achieved. Access may be accomplished by open surgery, small incision surgery, or percutaneously.

The surgical procedure chosen, planned, or performed will be for repair of bone fracture, repair of osteotomy, bone fusion, or other procedures in which two or more bones or bone portions must be held in close proximity to each other to facilitate bone healing, and in particular to facilitate bone fusion.

The patient's bone or bone portions are exposed and reduced to the desired positions.

Plate 300 is positioned such that recess 360 straddles a gap between two adjacent bones or bone portions.

Optionally a drill guide is inserted into fastener hole 12 of translating portion 390 and a hole is drilled into the patient's bone

Translating portion 390 is loosely affixed to at least one bone or bone portion by means of inserting at least one fastener 40 such as a bone screw into the fastener hole 12 of translating portion 390 and into the drilled hole (if any) then advancing the fastener into the patient's bone.

Any gap between the bone or bone portions immediately underlying plate 300 is reduced and a second fastener 40 such as a bone screw is inserted into hole 12 of locking portion 380, into a drilled hole (if any) and then advanced into the patient's bone causing the bone portions or bones to be locked together in a reduced configuration.

The bone or bone portions are further advanced towards each other to reduce any gap therebetween. This is allowed by system 300 due to the allowable longitudinal motion between translating portion and fixed portion. Fastener 40 on translating portion is immediately advanced to lock translating portion and adjacent locking portion of plate to the bone or bone portions.

Bone staple 30 is placed through recess 360 and through hole(s) 370, then into the bone or bone portions and the staple is allowed to hold the bone or bone portions at the desired positions relative to each other. The staple ends apply active compression forces against the bone at a distance below the bone plate distal surface.

In an alternate method staple 30 can be placed into the bone followed by placement of bone plate 310 and use of the translating aspect of system 300.

In another method of surgery, active compression is achieved by first, exposing the bone surface. Next, an orthopedic plate 310 having a translation portion 390 having a translation portion fastener hole and a locking portion having a locking portion fastener hole is placed on the bone, and fastened to the bone with a first fastener and a second fastener, and optionally a third fastener. It should be noted, that either the second or the third fastener is a compression fastener and the first fastener is a locking fastener. Then, the first fastener is locked in the locking portion fastener hole to form a locked relationship and a second fastener is placed in the translation portion fastener hole and the compression fastener drives active compression in the translation portion 360.

## Claims

1. A bone plate assembly (200,300) comprising:
a plate fixed member (210,310) having a first longitudinal axis (LA), a first screw hole (12) disposed near a plate fixed member first end portion, a first surface and a second, opposing bone facing surface, the plate first surface having a recess (260,360) near a plate fixed member distal end that accommodates a staple bridge (32), the plate (210,310) further including a first through-hole (270,370) that accommodates a staple first leg (31) and a second through-hole (270,370) that accommodates a staple second leg (31), and
a plate translation member (290,390) having a second longitudinal axis and a second screw hole (12) disposed near a translation member (290,390) second end portion,
wherein the plate translation member second end portion is movably coupled to the plate fixed member first end portion.

2. The bone plate assembly (200,300) as set forth in claim 1, wherein the plate translation member (290,390) permits the second screw hole (12) to be translated across the second longitudinal axis.

3. The bone plate assembly (200,300) as set forth in claim 1, wherein the second screw hole (12) is a threaded locking screw hole.

4. The bone plate assembly (200,300) as set forth in claim 1, wherein the first longitudinal axis (LA) and the second longitudinal axis are parallel.

5. The bone plate assembly (200,300) as set forth in claim 1, wherein the first longitudinal axis (LA) and the second longitudinal axis (LA) are the same axis.

6. The bone plate assembly (200,300) as set forth in claim 1, wherein the first and second screw holes (12) are disposed along the first longitudinal axis (LA).

7. The bone plate assembly (200,300) as set forth in claim 1, wherein the translation member (290,390) is moveable along at least one of the first longitudinal axis (LA) or the second longitudinal axis, relative to the fixed member (210,310).

8. The bone plate assembly (200,300) as set forth in claim 1, wherein at least one of the first through-hole (270,370) and the second through-hole (270,370) include internal threads.

9. The bone plate assembly (200,300) as set forth in claim 1, further comprising a bone staple (30) comprising a bridge (32) having a longitudinal axis, the bone staple (30) further having a first surface and a second surface and first leg (31) and a second leg (31) extending outward from bridge longitudinal axis at an angle of from 45° to 135 °.

10. The bone plate assembly (200,300) as set forth in claim 1, wherein the first through-hole (270,370) is at least partially threaded and the second through-hole (270,370) include a compression ramp (17) having an internal shoulder that slopes downward in a direction away from the first through-hole (270,370).

11. The bone plate assembly (200,300) as set forth in claim 1, wherein the recess (260,360) defines an intermediate surface facing the direction of the first plate surface and the bridge second surface contacts the intermediate surface in use.

12. The bone plate assembly (200,300) as set forth in claim 1, wherein the plate (210,310) further includes a compression slot (17b) having an edge defining a shoulder that slopes toward the second bone facing surface.
